# EUROPEAN PATENT APPLICATION

(11) **EP 4 557 425 A1**
(43) Date of publication of application: **21.05.2025**
(21) Application number: 24212399.0
(22) Date of filing: 12.11.2024
(51) Int. Cl.: H01M 10/0525, H01M 10/0567

(54) **ELECTROLYTE SOLUTION FOR RECHARGEABLE LITHIUM BATTERY AND RECHARGEABLE LITHIUM BATTERY INCLUDING THE SAME**

(30) Priority: 14.11.2023 KR 20230157629
(71) Applicant: SAMSUNG SDI CO., LTD., Yongin-si, Gyeonggi-do 17084 (KR)
(72) Inventor: Lee, Tae Jin, 17084 Yongin-si, Gyeonggi-do (KR); Kim, Minseo, 17084 Yongin-si, Gyeonggi-do (KR); Kim, Dahyun, 17084 Yongin-si, Gyeonggi-do (KR); Woo, Myungheui, 17084 Yongin-si, Gyeonggi-do (KR)
(74) Representative: Gulde & Partner

(57) **Abstract**

An electrolyte solution for a rechargeable lithium battery includes a non-aqueous organic solvent, a lithium salt; and an additive, wherein the additive includes a first compound represented by Chemical Formula 1 and a second compound represented by Chemical Formula 2.

## Description

### BACKGROUND

### 1. Field

An electrolyte solution for a rechargeable lithium battery, and a rechargeable lithium battery including the electrolyte solution, are disclosed.

### 2. Description of the Related Art

With increasing use of electronic devices that use batteries, such as, e.g., mobile phones, laptop computers, electric vehicles, and the like, the demand for rechargeable batteries with high energy density and high capacity is growing.

Rechargeable lithium batteries typically include a positive electrode and a negative electrode including an active material capable of intercalating and deintercalating lithium ions, and an electrolyte solution, and generate electrical energy through oxidation and reduction reactions when lithium ions are intercalated and deintercalated from the positive electrode and the negative electrode.

Rechargeable lithium batteries with high capacity, high energy density, and high safety for use as a driving power source for hybrid vehicles or electric vehicles, or as a power storage power source, may be advantageous.

In rechargeable lithium batteries, the electrolyte solution plays a role in transferring lithium ions, and can exhibit significantly higher ionic conductivity by including organic solvents and lithium salts. These electrolyte solutions play a role in determining the safety and performance of rechargeable lithium batteries.

When rechargeable lithium batteries are exposed to overcharging conditions or high temperatures, the rechargeable lithium batteries may rapidly generate heat and generate gas, leading to safety concerns such as, e.g., cell explosions.

Therefore, the development of an electrolyte solution with desired or improved safety even under overcharging and heat exposure conditions may be advantageous.

### SUMMARY

The invention is defined by the appended claims. The description that follows is subjected to this limitation. Any disclosure lying outside the scope of said claims is only intended for illustrative as well as comparative purposes.

Some example embodiments include an electrolyte solution for a rechargeable lithium battery exhibiting desired or improved safety under overcharge and heat exposure conditions.

Some example embodiments include a rechargeable lithium battery including the electrolyte solution.

An electrolyte solution for a rechargeable lithium battery includes a non-aqueous organic solvent, a lithium salt, and an additive, wherein the additive includes a first compound represented by Chemical Formula 1 and a second compound represented by Chemical Formula 2.

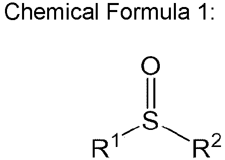

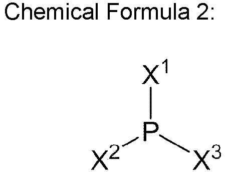

In Chemical Formula 1, R¹ and R² each independently is a substituted or unsubstituted C1 to C20 alkyl group or a substituted or unsubstituted C6 to C30 aryl group, provided that at least one of R¹ and R² is a substituted or unsubstituted C6 to C30 aryl group,

in Chemical Formula 2, X¹ to X³ each is independently a halogen or -O-L^{a}-R^{a},
at least one of X¹ to X³ is -O-L^{a}-R^{a},
L^{a} each independently is a single bond or a substituted or unsubstituted C1 to C10 alkylene group,
R^{a} each independently is at least one of a cyano group (-CN), a difluorophosphite group (-OPF₂), a substituted or unsubstituted C1 to C10 alkyl group, a substituted or unsubstituted C2 to C10 alkenyl group, a substituted or unsubstituted C2 to C10 alkynyl group, a substituted or unsubstituted C3 to C10 cycloalkyl group, a substituted or unsubstituted C3 to C10 cycloalkenyl group, a substituted or unsubstituted C3 to C10 cycloalkynyl group, or a substituted or unsubstituted C6 to C20 aryl group, and
R^{a} is each independently present, or at least two R^{a}s are linked to form a substituted or unsubstituted monocyclic or polycyclic aliphatic heterocycle, or a substituted or unsubstituted monocyclic or polycyclic aromatic heterocycle.

In some example embodiments, a rechargeable lithium battery includes a positive electrode including a positive electrode active material, a negative electrode including a negative electrode active material, a separator between the positive electrode and the negative electrode, and the aforementioned electrolyte solution.

An electrolyte solution for a rechargeable lithium battery according to some example embodiments may result in a battery having desired or improved safety under overcharge and heat exposure.

### BRIEF DESCRIPTION OF THE DRAWINGS

FIGS. 1 to 4 are schematic views of a rechargeable lithium battery, according to some example embodiments.
FIG. 5 is a graph showing the results of evaluating overcharge of the rechargeable lithium battery cells manufactured in Examples 1 to 2, and Comparative Examples 1 to 4.
FIG. 6 is a graph showing the results of heat exposure evaluation in rechargeable lithium battery cells manufactured in Examples 1 to 2, and Comparative Examples 1 to 4.

### DETAILED DESCRIPTION

Hereinafter, example embodiments of the present disclosure will be described in detail. However, these example embodiments are presented as an example, and the present disclosure is not limited thereto, and the present disclosure is defined by the scope of the claims described below.

As used herein, when specific definition is not otherwise provided, it will be understood that when an element such as a layer, film, region, or substrate is referred to as being "on" another element, it can be directly on the other element or intervening elements may also be present.

Unless otherwise specified in this specification, what is indicated in the singular may also include the plural.

As used herein, "combination thereof" indicates a mixture, laminate, composite, copolymer, alloy, blend, reaction product, and the like of the constituents.

As used herein, when a definition is not otherwise provided, a particle diameter may be an average particle diameter. In addition, the particle diameter indicates the average particle diameter (D50), which is the diameter of particles having a cumulative volume of 50 volume% in the particle size distribution. The average particle size (D50) may be measured by a method known to those skilled in the art, for example, by a particle size analyzer, or by a transmission electron microscope image, or a scanning electron microscope image. Alternatively, a dynamic light-scattering measurement device is used to perform a data analysis, and the number of particles is counted for each particle size range. From this, the average particle diameter (D50) value may be easily obtained through a calculation. Alternatively, it can be measured using a laser diffraction method. When measuring by the laser diffraction method, for example, the particles to be measured are dispersed in a dispersion medium, and then introduced into a commercially available laser diffraction particle diameter measuring device (e.g., Microtrac MT 3000), and ultrasonic waves of about 28 kHz with an output of 60 Ware irradiated to calculate an average particle diameter (D50) on the basis of 50% of the particle diameter distribution in the measuring device.

As used herein, when a definition is not otherwise provided, "substituted" refers to replacement of at least one hydrogen of a substituent or a compound by deuterium, a halogen, a hydroxyl group, an amino group, a C1 to C30 amine group, a nitro group, a C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 fluoroalkyl group, or a cyano group.

For example, "substituted" may refer to replacement of at least one hydrogen of a substituent or a compound by deuterium, a halogen, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C10 fluoroalkyl group, or a cyano group. For example, "substituted" may refer to replacement of at least one hydrogen of a substituent or a compound by deuterium, a halogen, a C1 to C20 alkyl group, a C6 to C30 aryl group, a C1 to C10 fluoroalkyl group, or a cyano group. Alternatively, "substituted" may refer to replacement of at least one hydrogen of a substituent or a compound by deuterium, a halogen group, a C1 to C5 alkyl group, a C6 to C18 aryl group, a C1 to C5 fluoroalkyl group, or a cyano group. For example, "substituted" may refer to replacement of at least one hydrogen of a substituent or a compound by deuterium, a cyano group, a halogen, a methyl group, an ethyl group, a propyl group, a butyl group, a phenyl group, a biphenyl group, a terphenyl group, a trifluoromethyl group, or a naphthyl group.

An electrolyte solution for a rechargeable lithium battery according to some example embodiments includes a non-aqueous organic solvent; a lithium salt; and an additive, wherein the additive includes a first compound, and a second compound. The first and second compounds are described in detail below.

When the first compound and the second compound are used in combination, both stability of the battery under overcharge and safety of the battery under heat exposure can be effectively achieved.

### First Compound

The first compound is a sulfoxide-based compound, which effectively reduces or suppresses the heating temperature of the battery under overcharge operating conditions.

The first compound is represented by Chemical Formula 1.

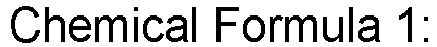

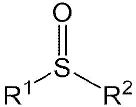

In Chemical Formula 1, R¹ and R² each independently is a substituted or unsubstituted C1 to C20 alkyl group or a substituted or unsubstituted C6 to C30 aryl group, provided that at least one of R¹ and R² is a substituted or unsubstituted C6 to C30 aryl group.

In some example embodiments, Chemical Formula 1 may be represented by Chemical Formula 1-1 or Chemical Formula 1-2. As an example, Chemical Formula 1 may be represented by Chemical Formula 1-1.

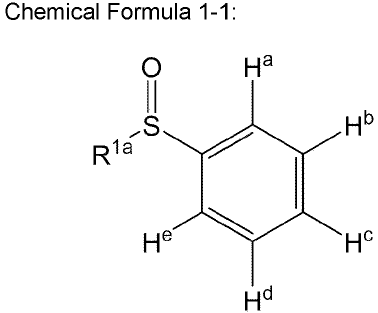

In Chemical Formula 1-1,
R^{1a} is a substituted or unsubstituted C1 to C20 alkyl group, and
H^{a} to H^{e} are each independently at least one of hydrogen, a halogen, a hydroxyl group, a cyano group, a nitro group, a substituted or unsubstituted C1 to C20 alkyl group, a substituted or unsubstituted C1 to C20 alkoxy group, a substituted or unsubstituted C2 to C20 alkenyl group, a substituted or unsubstituted C2 to C20 alkynyl group, a substituted or unsubstituted C3 to C20 cycloalkyl group, a substituted or unsubstituted C6 to C20 aryl group, or a substituted or unsubstituted C2 to C20 heteroaryl group.

As an example, H^{a} to H^{e} may each independently be at least one of hydrogen, a halogen group, a substituted or unsubstituted C1 to C20 alkyl group, or a substituted or unsubstituted C1 to C20 alkoxy group.

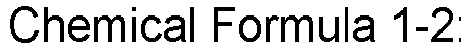

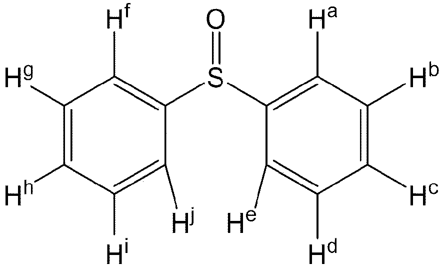

In Chemical Formula 1-2,
H^{a} to H^{j} are each independently at least one of hydrogen, a halogen, a hydroxyl group, a cyano group, a nitro group, a substituted or unsubstituted C1 to C20 alkyl group, a substituted or unsubstituted C1 to C20 alkoxy group, a substituted or unsubstituted C2 to C20 alkenyl group, a substituted or unsubstituted C2 to C20 alkynyl group, a substituted or unsubstituted C3 to C20 cycloalkyl group, a substituted or unsubstituted C6 to C20 aryl group, or a substituted or unsubstituted C2 to C20 heteroaryl group.

As an example, H^{a} to H^{j} may each independently be or include at least one of hydrogen, a halogen group, a substituted or unsubstituted C1 to C20 alkyl group, or a substituted or unsubstituted C1 to C20 alkoxy group.

As an example, the first compound may be any one or more of the compounds listed in Group 1.

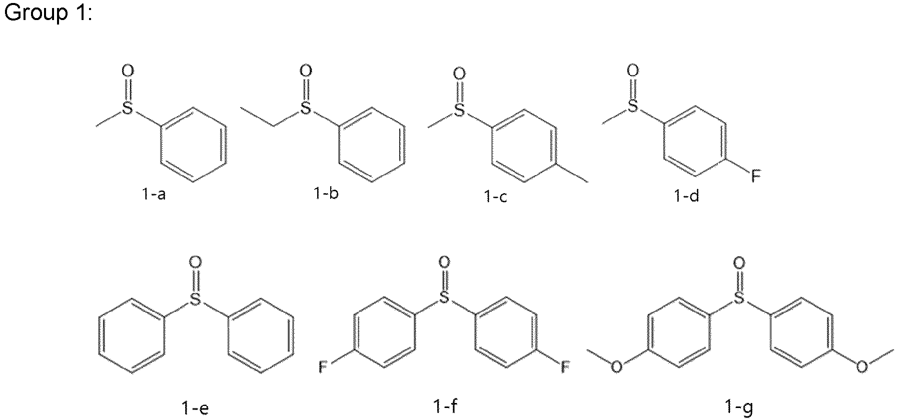

In some example embodiments, the first compound may be included in an amount that is greater than 0.05 wt%, or greater than or equal to 0.1 wt%, and less than 6 wt%, or less than or equal to 5 wt%, based on a total weight of the electrolyte solution for the rechargeable lithium battery.

As an example, the first compound may be included in an amount that is greater than 0.05 wt% and less than 6 wt%, for example, greater than 0.05 wt% and less than or equal to 5 wt%, greater than or equal to 0.1 wt% and less than 6 wt%, or 0.1 wt% to 5 wt%.

When the first compound is included in an amount that is less than or equal to 0.05 wt% based on a total weight of the electrolyte solution for a rechargeable lithium battery, improving battery safety during overcharging may be minimal, and when first compound is included in an amount that is greater than or equal to 6 wt%, the resistance of the battery may increase excessively.

### Second Compound

The second compound is configured to stabilize the lithium salt within the electrolyte solution, thereby hindering or preventing the electrolyte solution from decomposing at high temperatures. Accordingly, gas generation inside the battery at high temperatures is effectively reduced or suppressed, so that battery safety and cycle-life characteristics may be simultaneously or contemporaneously improved under heat exposure.

The second compound may be represented by Chemical Formula 2.

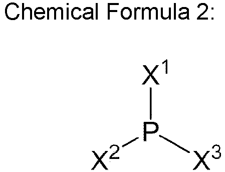

In Chemical Formula 2, X¹ to X³ each independently are a halogen or -O-L^{a}-R^{a},
at least one of X¹ to X³ is -O-L^{a}-R^{a},
L^{a} each independently is a single bond or a substituted or unsubstituted C1 to C10 alkylene group,
R^{a} each independently is at least one of a cyano group (-CN), a difluorophosphite group (-OPF₂), a substituted or unsubstituted C1 to C10 alkyl group, a substituted or unsubstituted C2 to C10 alkenyl group, a substituted or unsubstituted C2 to C10 alkynyl group, a substituted or unsubstituted C3 to C10 cycloalkyl group, a substituted or unsubstituted C3 to C10 cycloalkenyl group, a substituted or unsubstituted C3 to C10 cycloalkynyl group, or a substituted or unsubstituted C6 to C20 aryl group, and
R^{a} is each independently present, or at least two R^{a}s are linked to form a substituted or unsubstituted monocyclic or polycyclic aliphatic heterocycle, or a substituted or unsubstituted monocyclic or polycyclic aromatic heterocycle.

In an example, the substituted or unsubstituted monocyclic or polycyclic aliphatic heterocycle may be or include a C1 to C8 heterocycloalkyl group including a heteroatom of N, O, S, P or Si in the ring, and the substituted or unsubstituted aromatic heterocycle may be or include a C3 to C8 heteroaryl group including a heteroatom of N, O, S, P or Si in the ring.

In some example embodiments, Chemical Formula 2 may be represented by any one of Chemical Formula 2-1 to Chemical Formula 2-3.

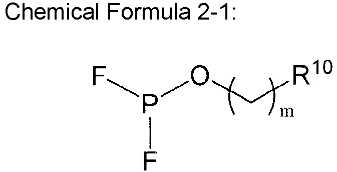

In Chemical Formula 2-1,
m is an integer in a range of 1 to 5, and
R¹⁰ is a cyano group (-CN) or a difluorophosphite group (-OPF₂).

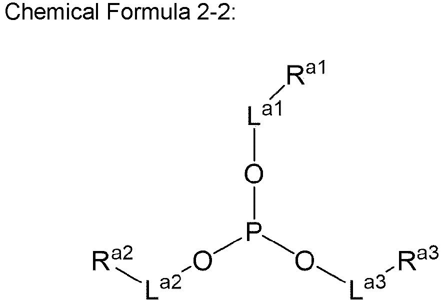

In Chemical Formula 2-2,
L^{a1} to L^{a3} are each independently a single bond or a substituted or unsubstituted C1 to C5 alkylene group, and
R^{a1} to R^{a3} are each independently at least one of a substituted or unsubstituted C1 to C10 alkyl group, a substituted or unsubstituted C2 to C10 alkenyl group, a substituted or unsubstituted C2 to C10 alkynyl group, a substituted or unsubstituted C3 to C10 cycloalkyl group, a substituted or unsubstituted C3 to C10 cycloalkenyl group, a substituted or unsubstituted C3 to C10 cycloalkynyl group, or a substituted or unsubstituted C6 to C20 aryl group.

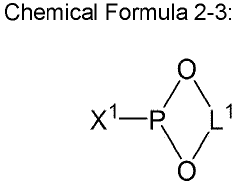

In Chemical Formula 2-3,
X¹ is a halogen or -O-L^{a4}-R^{a4},
L^{a4} is a single bond or a substituted or unsubstituted C1 to C5 alkylene group,
R^{a4} is at least one of a substituted or unsubstituted C1 to C10 alkyl group, a substituted or unsubstituted C2 to C10 alkenyl group, a substituted or unsubstituted C2 to C10 alkynyl group, a substituted or unsubstituted C3 to C10 cycloalkyl group, a substituted or unsubstituted C3 to C10 cycloalkenyl group, a substituted or unsubstituted C3 to C10 cycloalkynyl group, or a substituted or unsubstituted C6 to C20 aryl group, and
L¹ is a substituted or unsubstituted C2 to C5 alkylene group.

For example, the second compound may be or include at least one of the compounds listed in Group 2.

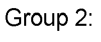

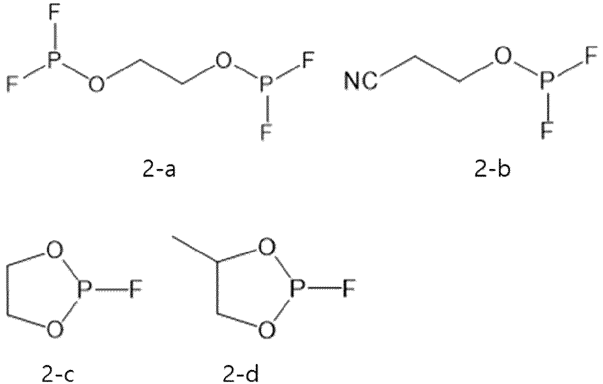

According to an example embodiment, the additive included in the electrolyte solution for a rechargeable lithium battery may be or include a composition including at least one of the compounds listed in Group 1 as the first compound and at least one of the compounds listed in Group 2 as the second compound.

For example, an additive included in an electrolyte solution for a rechargeable lithium battery may be or include a composition including Compound 1-a of Group 1 as a first compound, and Compound 2-a or Compound 2-d as a second compound.

In some example embodiments, the second compound may be included in an amount that is greater than 0.05 wt%, or greater than or equal to 0.1 wt%, and less than 5 wt%, less than 4 wt%, or less than or equal to 3 wt%, based on a total weight of the electrolyte solution for the rechargeable lithium battery.

As an example, the second compound may be included in an amount that is greater than 0.05 wt% and less than 4 wt%, or 0.1 wt% to 3 wt%, based on a total weight of the electrolyte solution for a rechargeable lithium battery.

When the second compound is included in an amount that is less than or equal to 0.05 wt% based on a total weight of the electrolyte solution for a rechargeable lithium battery, improving battery safety during heat exposure is minimal, and when the second compound is included in an amount that is greater than or equal to 4 wt%, the resistance of the battery may excessively increase.

In some example embodiments, the first compound and the second compound may be included in a weight ratio in a range of 0.01:1 to 100:1, for example, a weight ratio of 0.05:1 to 100:1, a weight ratio of 0.01:1 to 40:1, a weight ratio of 0.05:1 to 40:1, a weight ratio of 0.05:1 to 20:1, or a weight ratio of 0.1:1 to 20:1.

When the weight ratio of the first compound and the second compound satisfies the above numerical range, a battery having desired or improved safety under both overcharge and heat exposure can be obtained.

The electrolyte solution for a rechargeable lithium battery includes a non-aqueous organic solvent and a lithium salt.

The non-aqueous organic solvent may constitute a medium for transmitting ions taking part in the electrochemical reaction of a battery.

The non-aqueous organic solvent may be or include at least one of a carbonate-based, ester-based, ether-based, ketone-based, or alcohol-based solvent, an aprotic solvent, or a combination thereof.

The carbonate-based solvent may include at least one of dimethyl carbonate (DMC), diethyl carbonate (DEC), dipropyl carbonate (DPC), methylpropyl carbonate (MPC), ethylpropyl carbonate (EPC), methylethyl carbonate (MEC), ethylene carbonate (EC), propylene carbonate (PC), butylene carbonate (BC), and the like. The ester-based solvent may include at least one of methyl acetate, ethyl acetate, n-propyl acetate, dimethylacetate, methylpropionate, ethyl propionate, decanolide, mevalonolactone, valerolactone, caprolactone, and the like. The ether-based solvent may include at least one of dibutyl ether, tetraglyme, diglyme, dimethoxyethane, 2-methyltetrahydrofuran, 2,5-dimethyltetrahydrofuran, tetrahydrofuran, and the like. In addition, the ketone-based solvent may include cyclohexanone, and the like. The alcohol-based solvent may include at least one of ethanol, isopropyl alcohol, and the like and the aprotic solvent may include at least one of nitriles such as R-CN (wherein R is a C2 to C20 linear, branched, or cyclic hydrocarbon group, a double bond, an aromatic ring, or an ether bond, and the like; amides such as dimethylformamide; dioxolanes such as 1,3-dioxolane, 1,4-dioxolane, and the like; sulfolanes, and the like.

The non-aqueous organic solvents may be used alone or in combination of two or more solvents.

In addition, when the solvent is a carbonate-based solvent, a cyclic carbonate and a chain carbonate may be mixed, and the cyclic carbonate and the chain carbonate may be mixed in a volume ratio in a range of about 1:1 to about 1:9.

The lithium salt dissolved in the organic solvent is configured to supply lithium ions in a battery, to enable a basic operation of a rechargeable lithium battery, and to improve transportation of the lithium ions between positive and negative electrodes. For example, the lithium salt may include at least one of LiPF₆, LiBF₄, LiSbF₆, LiAsF₆, LiClO₄, LiAlO₂, LiAlCl₄, LiPO₂F₂, LiCI, Lil, LiN(SO₃C₂F₅)₂, Li(FSO₂)₂N (lithium bis(fluorosulfonyl)imide, LiFSI), LiC₄F₉SO₃, LiN(CₓF₂ₓ₊₁SO₂)(C_{y}F_{2y+1}SO₂), x and y are integers of 1 to 20, lithium trifluoromethane sulfonate, lithium tetrafluoroethanesulfonate, lithium difluorobis(oxalato)phosphate (LiDFOB), and lithium bis(oxalato) borate (LiBOB).

A rechargeable lithium battery according to some example embodiments includes a positive electrode including a positive electrode active material, a negative electrode including a negative electrode active material, a separator between the positive electrode and the negative electrode, and the aforementioned electrolyte solution.

### Positive Electrode Active Material

The positive electrode active material may include a compound (lithiated intercalation compound) capable of intercalating and deintercalating lithium. For example, at least one of a composite oxide of lithium and a metal such as or including at least one of cobalt, manganese, nickel, and combinations thereof may be used.

The composite oxide may be or include a lithium transition metal composite oxide, and examples thereof may include at least one of lithium nickel-based oxide, lithium cobalt-based oxide, lithium manganese-based oxide, lithium iron phosphate-based compound, cobalt-free lithium nickel-manganese-based oxide, or a combination thereof.

As an example, the following compounds represented by any one of the following chemical formulas may be used. LiₐA_{1-b}X_{b}O_{2-c}D_{c} (0.90≤a≤1.8, 0≤b≤0.5, and 0≤c≤0.05); LiₐMn_{2- b}X_{b}O_{4-c}D_{c} (0.90≤a≤1.8, 0≤b≤0.5, and 0≤c≤0.05); LiₐNi_{1-b-c}Co_{b}X_{c}O_{2-α}O_{α} (0.90≤a≤1.8, 0≤b≤0.5, 0≤c≤0.5, and 0<α<2); LiₐNi_{1-b-c}Mn_{b}X_{c}O_{2-α}O_{α} (0.90≤a≤1.8, 0≤b≤0.5, 0≤c≤0.5, and 0<α<2); LiₐNi_{b}Co_{c}L¹_{d}GₑO₂ (0.90≤a≤1.8, 0≤b≤0.9, 0≤c≤0.5, 0≤d≤0.5, and 0≤e≤0.1); LiₐNiG_{b}O₂ (0.90≤a≤1.8 and 0.001≤b≤0.1); LiₐCoG_{b}O₂ (0.90≤a≤1.8 and 0.001≤b≤0.1); LiₐMn_{1-b}G_{b}O₂ (0.90≤a≤1.8 and 0.001≤b≤0.1); LiₐMn₂G_{b}O₄ (0.90≤a≤1.8 and 0.001≤b≤0.1); LiₐMn_{1-g}G_{g}PO₄ (0.90≤a≤1.8, 0≤g≤0.5); Li_{(3-f)}Fe₂(PO₄)₃ (0≤f≤2); or LiₐFePO₄ (0.90≤a≤1.8).

In the above chemical formulas, A is or includes at least one of Ni, Co, Mn, or a combination thereof; X is or includes at least one of Al, Ni, Co, Mn, Cr, Fe, Mg, Sr, V, a rare earth element or a combination thereof; D is or includes at least one of O, F, S, P, or a combination thereof; G is or includes at least one of Al, Cr, Mn, Fe, Mg, La, Ce, Sr, V, or a combination thereof; and L¹ is or includes at least one of Mn, Al, or a combination thereof.

For example, the positive electrode active material may be or include a high nickel-based positive electrode active material having a nickel content of greater than or equal to about 80 mol%, greater than or equal to about 85 mol%, greater than or equal to about 90 mol%, greater than or equal to about 91 mol%, or greater than or equal to about 94 mol% and less than or equal to about 99 mol% based on 100 mol% of the metal excluding lithium in the lithium transition metal composite oxide. The high-nickel-based positive electrode active material can achieve high capacity and can be applied to a high-capacity, high-density rechargeable lithium battery.

### Positive Electrode

A positive electrode for a rechargeable lithium battery may include a current collector and a positive electrode active material layer on the current collector. The positive electrode active material layer may include a positive electrode active material and may further include a binder and/or a conductive material.

For example, the positive electrode may further include an additive that can constitute a sacrificial positive electrode.

An amount of the positive electrode active material may be in a range of about 90 wt% to about 99.5 wt% based on 100% by weight of the positive electrode active material layer, and amounts of the binder and the conductive material may be in a range of about 0.5 wt% to about 5 wt%, respectively, based on 100 wt% of the positive electrode active material layer.

The binder is configured to attach the positive electrode active material particles to each other, and to attach the positive electrode active material to the current collector. Examples of the binder may include at least one of polyvinyl alcohol, carboxymethylcellulose, hydroxypropylcellulose, diacetylcellulose, polyvinylchloride, carboxylated polyvinylchloride, polyvinylfluoride, a polymer including ethylene oxide, polyvinylpyrrolidone, polyurethane, polytetrafluoroethylene, polyvinylidene fluoride, polyethylene, polypropylene, a styrene-butadiene rubber, a (meth)acrylated styrene-butadiene rubber, an epoxy resin, a (meth)acrylic resin, a polyester resin, nylon, and the like, but are not limited thereto.

The conductive material may be configured to impart conductivity to the electrode, and any material that does not cause chemical change and conducts electrons can be used in the battery. Examples of the conductive material may include a carbon-based material such as or including at least one of natural graphite, artificial graphite, carbon black, acetylene black, ketjen black, a carbon fiber, a carbon nanofiber, and carbon nanotube; a metal-based material containing at least one of copper, nickel, aluminum, silver, etc., in a form of a metal powder or a metal fiber; a conductive polymer such as a polyphenylene derivative; or a mixture thereof.

Al may be used as the current collector, but is not limited thereto.

### Negative Electrode Active Material

The negative electrode active material may include at least one of a material that reversibly intercalates/deintercalates lithium ions, a lithium metal, a lithium metal alloy, a material capable of doping/dedoping lithium, or a transition metal oxide.

The material that reversibly intercalates/deintercalates lithium ions may include a carbon-based negative electrode active material, for example crystalline carbon, amorphous carbon or a combination thereof. The crystalline carbon may be or include graphite such as non-shaped, sheet-shaped, flake-shaped, sphere-shaped, or fiber-shaped natural graphite or artificial graphite, and the amorphous carbon may be or include at least one of a soft carbon, a hard carbon, a mesophase pitch carbonization product, calcined coke, and the like.

The lithium metal alloy includes an alloy of lithium and a metal such as or including at least one of Na, K, Rb, Cs, Fr, Be, Mg, Ca, Sr, Si, Sb, Pb, In, Zn, Ba, Ra, Ge, Al, and Sn.

The material capable of doping/dedoping lithium may be or include at least one of a Si-based negative electrode active material or a Sn-based negative electrode active material. The Si-based negative electrode active material may include silicon, a silicon-carbon composite, SiOx (0 < x < 2), a Si-Q alloy (wherein Q is or includes at least one of an alkali metal, an alkaline-earth metal, a Group 13 element, a Group 14 element (excluding Si), a Group 15 element, a Group 16 element, a transition metal, a rare earth element, and a combination thereof). The Sn-based negative electrode active material may include at least one of Sn, SnO₂, a Sn-based alloy, or a combination thereof.

The silicon-carbon composite may be or include a composite of silicon and amorphous carbon. According to an example embodiment, the silicon-carbon composite may be in the form of silicon particles and amorphous carbon coated on the surface of the silicon particles. For example, the silicon-carbon composite may include a secondary particle (core) in which primary silicon particles are assembled, and an amorphous carbon coating layer (shell) on the surface of the secondary particle. The amorphous carbon may also be between the primary silicon particles, and, for example, the primary silicon particles may be coated with the amorphous carbon. The secondary particles may be dispersed in an amorphous carbon matrix.

The silicon-carbon composite may further include crystalline carbon. For example, the silicon-carbon composite may include a core including crystalline carbon and silicon particles, and an amorphous carbon coating layer on a surface of the core.

The Si-based negative electrode active material or the Sn-based negative electrode active material may be used in combination with a carbon-based negative electrode active material.

### Negative Electrode

The negative electrode for a rechargeable lithium battery includes a current collector and a negative electrode active material layer on the current collector. The negative electrode active material layer may include a negative electrode active material, and may further include a binder and/or a conductive material.

For example, the negative electrode active material layer may include about 90 wt% to about 99 wt% of the negative electrode active material, about 0.5 wt% to about 5 wt% of the binder, and about 0 wt% to about 5 wt% of the conductive material.

The binder is configured to attach the negative electrode active material particles to each other, and to attach the negative electrode active material to the current collector. The binder may include a non-aqueous binder, an aqueous binder, a dry binder, or a combination thereof.

The non-aqueous binder may include at least one of polyvinyl chloride, carboxylated polyvinyl chloride, polyvinyl fluoride, an ethylene propylene copolymer, polystyrene, polyurethane, polytetrafluoroethylene, polyvinylidene fluoride, polyethylene, polypropylene, poly amideimide, polyimide, or a combination thereof.

The aqueous binder may be or include at least one of a styrene-butadiene rubber, a (meth)acrylated styrene-butadiene rubber, a (meth)acrylonitrile-butadiene rubber, (meth)acrylic rubber, a butyl rubber, a fluoro rubber, polyethylene oxide, polyvinylpyrrolidone, polyepichlorohydrin, polyphosphazene, poly(meth)acrylonitrile, an ethylene propylene diene copolymer, polyvinylpyridine, chlorosulfonated polyethylene, latex, a polyester resin, a (meth)acrylic resin, a phenol resin, an epoxy resin, polyvinyl alcohol, and a combination thereof.

When an aqueous binder is used as the negative electrode binder, a cellulose-based compound capable of imparting viscosity may be further included. The cellulose-based compound may include at least one of carboxymethyl cellulose, hydroxypropylmethyl cellulose, methyl cellulose, or an alkali metal salt thereof. The alkali metal may include Na, K, or Li.

The dry binder may be or include a polymer material capable of being fibrous, and may be or include, for example, at least one of polytetrafluoroethylene, polyvinylidene fluoride, a polyvinylidene fluoride-hexafluoropropylene copolymer, polyethylene oxide, or a combination thereof.

The conductive material may be configured to impart conductivity to the electrode, and any material that does not cause chemical change and conducts electrons can be used in the battery. Examples thereof may include a carbon-based material such as or including at least one of natural graphite, artificial graphite, carbon black, acetylene black, ketjen black, a carbon fiber, a carbon nanofiber, and a carbon nanotube; a metal-based material including copper, nickel, aluminum, silver, etc. in a form of a metal powder or a metal fiber; a conductive polymer such as a polyphenylene derivative; or a mixture thereof.

The negative electrode current collector may include at least one of a copper foil, a nickel foil, a stainless steel foil, a titanium foil, a nickel foam, a copper foam, a polymer substrate coated with a conductive metal, and a combination thereof.

### Separator

Depending on the type of the rechargeable lithium battery, a separator may be present between the positive electrode and the negative electrode. The separator may include at least one of polyethylene, polypropylene, polyvinylidene fluoride, or a multilayer film of two or more layers thereof, and a mixed multilayer film such as a polyethylene/polypropylene two-layer separator, polyethylene/polypropylene/polyethylene three-layer separator, polypropylene/polyethylene/polypropylene three-layer separator, and the like.

The separator may include a porous substrate, and a coating layer including an organic material, an inorganic material, or a combination thereof on one or both surfaces of the porous substrate.

The porous substrate may be or include a polymer film such as or including at least one of polyolefin such as polyethylene and polypropylene, polyester such as polyethylene terephthalate and polybutylene terephthalate, polyacetal, polyamide, polyimide, polycarbonate, polyether ketone, polyarylether ketone, polyether ketone, polyetherimide, polyamideimide, polybenzimidazole, polyethersulfone, polyphenylene oxide, a cyclic olefin copolymer, polyphenylene sulfide, polyethylene naphthalate, a glass fiber, TEFLON, and polytetrafluoroethylene, or a copolymer or mixture of two or more thereof.

The organic material may include a polyvinylidene fluoride-based polymer or a (meth)acrylic polymer.

The inorganic material may include inorganic particles such as or including at least one of Al₂O₃, SiO₂, TiO₂, SnO₂, CeO₂, MgO, NiO, CaO, GaO, ZnO, ZrO₂, Y₂O₃, SrTiO₃, BaTiO₃, Mg(OH)₂, boehmite, and a combination thereof, but is not limited thereto.

The organic material and the inorganic material may be mixed in one coating layer, or a coating layer including an organic material and a coating layer including an inorganic material may be stacked.

### Rechargeable Lithium Battery

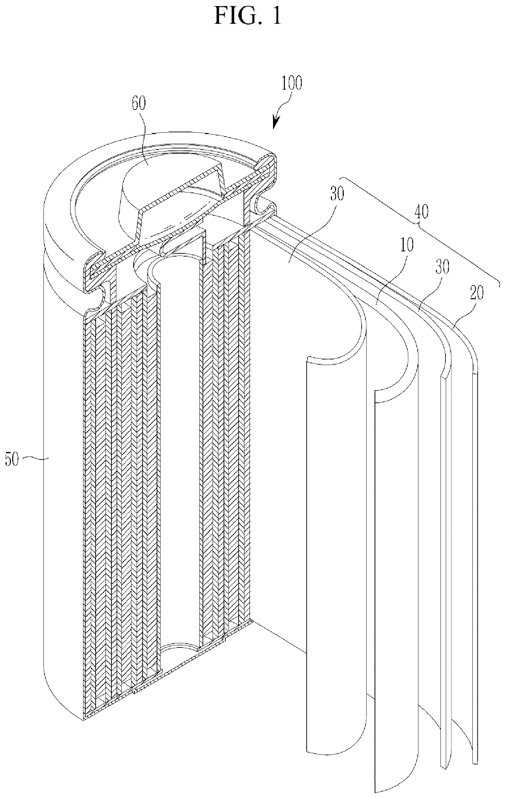

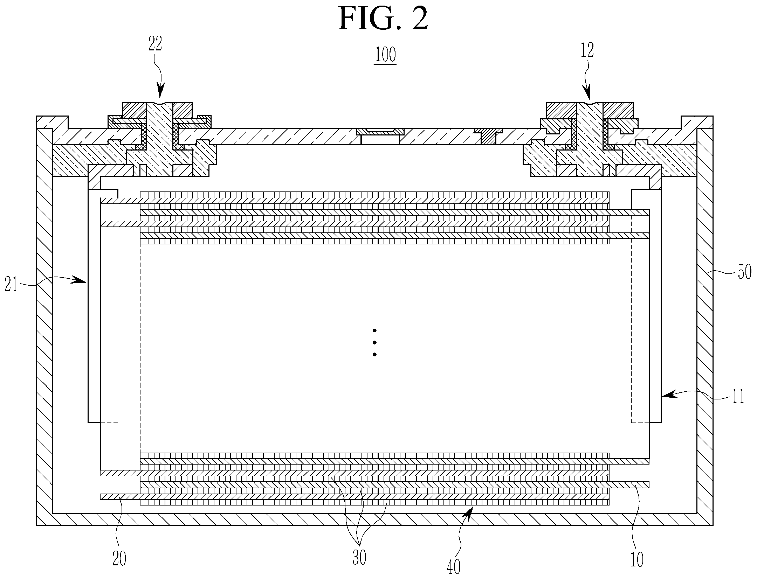

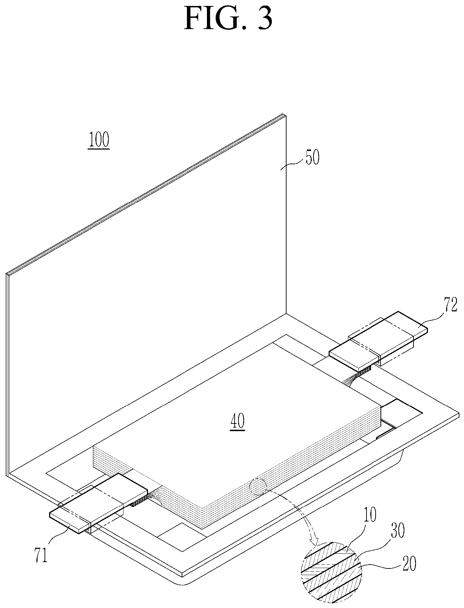

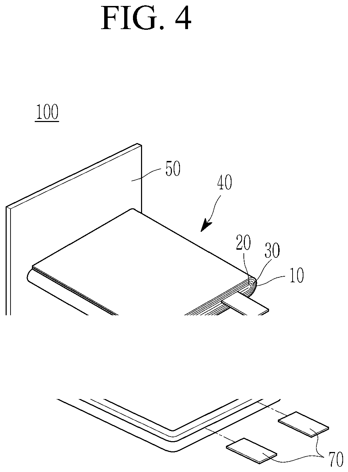

The rechargeable lithium battery may be classified into cylindrical, prismatic, pouch, coin, and the like depending on their shape. FIGS. 1 to 4 are schematic views illustrating a rechargeable lithium battery according to some example embodiments. FIG. 1 illustrates a cylindrical battery, FIG. 2 illustrates a prismatic battery, and FIGS. 3 and 4 illustrate pouch-type batteries. Referring to FIGS. 1 to 4, the rechargeable lithium battery 100 includes an electrode assembly 40 including a separator 30 between a positive electrode 10 and a negative electrode 20, and a case in which the electrode assembly 40 is included. The positive electrode 10, the negative electrode 20, and the separator 30 may be impregnated with an electrolyte solution (not shown). The rechargeable lithium battery 100 may include a sealing member 60 that seals the case 50 as shown in FIG. 1. Additionally, in FIG. 2, the rechargeable lithium battery 100 may include a positive electrode lead tab 11, a positive electrode terminal 12, a negative lead tab 21, and a negative electrode terminal 22. As shown in FIGS. 3 and 4, the rechargeable lithium battery 100 includes an electrode tab 70 illustrated in FIG. 4, and a positive electrode tab 71 and a negative electrode tab 72 illustrated in FIG. 3, the electrode tabs 70/71/72 forming an electrical path for inducing the current formed in the electrode assembly 40 to the outside of the battery 100.

The rechargeable lithium battery according to an example embodiment may be applicable to automobiles, mobile phones, and/or various types of electric devices, but the present disclosure is not limited thereto.

Examples and comparative examples of the present disclosure are described below. However, the following are only examples of the present disclosure, and the present disclosure is not limited to the following examples.

### Examples:

### Example 1

1.15 M LiPF₆ lithium salt is dissolved in a non-aqueous organic solvent mixed with ethylene carbonate (EC), methyl ethyl carbonate (MEC), and diethyl carbonate (DEC) in a volume ratio of 20:10:70 to prepare a basic electrolyte solution.

An electrolyte solution is prepared by adding Compound 1-a as a first compound and Compound 2-a as a second compound to the basic electrolyte solution.

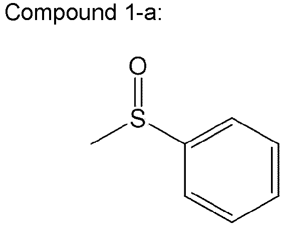

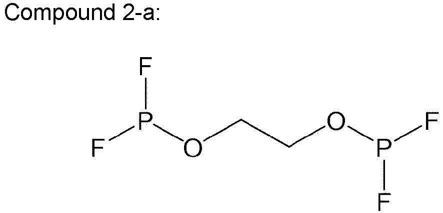

Herein, 2 wt% of the first compound and 1 wt% of the second compound are added based on a total amount that is the electrolyte solution.

LiNi_{0.91}Co_{0.07}Al_{0.02}O₂ as a positive electrode active material, polyvinylidene fluoride as a binder, and ketjen black as a conductive material are mixed in a weight ratio of 97:2:1 and dispersed in N-methyl pyrrolidone, preparing positive electrode active material slurry.

The positive electrode active material slurry is coated in a 14 µm-thick Al foil, dried at 110 °C, and compressed, manufacturing a positive electrode.

A negative electrode active material slurry is prepared by mixing artificial graphite as a negative electrode active material, styrene-butadiene rubber as a binder, and carboxylmethyl cellulose as a thickener in a weight ratio of 97:1:2 and dispersing the mixture in distilled water. The negative electrode active material slurry is coated on a 10 µm-thick Cu foil current collector, dried at 100 °C, and compressed, manufacturing a negative electrode.

Between the positive electrode and the negative electrode, a 25 µm-thick separator with a polyethylene-polypropylene multi-layer structure is interposed to manufacture an electrode assembly, and a rechargeable lithium battery cell is manufactured by inserting the electrode assembly into a cylindrical battery case and injecting the prepared electrolyte solution thereinto.

### Example 2

A rechargeable lithium battery cell is manufactured in the same manner as in Example 1, with a difference that Compound 2-d is used as the second compound in manufacturing the electrolyte solution.

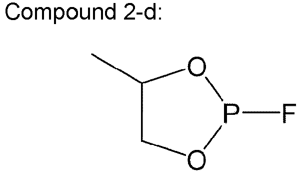

### Examples 3 to 6

A rechargeable lithium battery cell is manufactured in the same manner as in Example 1, with a difference that the contents of the first compound are 0.05 wt%, 0.1 wt%, 5 wt%, and 6 wt%, respectively, based on the total electrolyte solution.

### Examples 7 to 10

A rechargeable lithium battery cell is manufactured in the same manner as in Example 2, with a difference that the contents of the first compound are 0.05 wt%, 0.1 wt%, 5 wt%, and 6 wt%, respectively, based on the total electrolyte solution.

### Examples 11 to 14

A rechargeable lithium battery cell is manufactured in the same manner as in Example 1, with a difference that the contents of the second compound are 0.05 wt%, 0.1 wt%, 3 wt%, and 4 wt%, respectively, based on the total electrolyte solution.

### Examples 15 to 18

A rechargeable lithium battery cell is manufactured in the same manner as in Example 2, with a difference that the contents of the second compound are 0.05 wt%, 0.1 wt%, 3 wt%, and 4 wt%, respectively, based on the total electrolyte solution.

### Comparative Example 1

A rechargeable lithium battery cell is manufactured in the same manner as in Example 1, with a difference that the first compound and the second compound are not added to the electrolyte solution.

### Comparative Example 2

A rechargeable lithium battery cell is manufactured in the same manner as in Example 1, with a difference that the second compound is not added when preparing the electrolyte solution.

### Comparative Example 3

A rechargeable lithium battery cell is manufactured in the same manner as in Example 1, with a difference that the first compound is not added when preparing the electrolyte solution.

### Comparative Example 4

A rechargeable lithium battery cell is manufactured in the same manner as in Example 2, with a difference that the first compound is not added when preparing the electrolyte solution.

The types and contents of the first compound and the second compound included in the electrolyte solutions of Examples 1 to 18 and Comparative Examples 1 to 4 manufactured above are shown in Table 1 below.

**Table 1:**

| | First compound (wt%) (Compound 1-a) | Second compound (wt%) | |
|---|---|---|---|
| | | Compound 2-a | Compound 2-d |
| Comparative Example 1 | 0 | 0 | 0 |
| Comparative Example 2 | 2 | 0 | 0 |
| Comparative Example 3 | 0 | 1 | 0 |
| Comparative Example 4 | 0 | 0 | 1 |
| Example 1 | 2 | 1 | 0 |
| Example 2 | 2 | 0 | 1 |
| Example 3 | 0.05 | 1 | 0 |
| Example 4 | 0.1 | 1 | 0 |
| Example 5 | 5 | 1 | 0 |
| Example 6 | 6 | 1 | 0 |
| Example 7 | 0.05 | 0 | 1 |
| Example 8 | 0.1 | 0 | 1 |
| Example 9 | 5 | 0 | 1 |
| Example 10 | 6 | 0 | 1 |
| Example 11 | 2 | 0.05 | 0 |
| Example 12 | 2 | 0.1 | 0 |
| Example 13 | 2 | 3 | 0 |
| Example 14 | 2 | 4 | 0 |
| Example 15 | 2 | 0 | 0.05 |
| Example 16 | 2 | 0 | 0.1 |
| Example 17 | 2 | 0 | 3 |
| Example 18 | 2 | 0 | 4 |

### Evaluation Examples

### Evaluation Example 1: Evaluation of Overcharge Safety

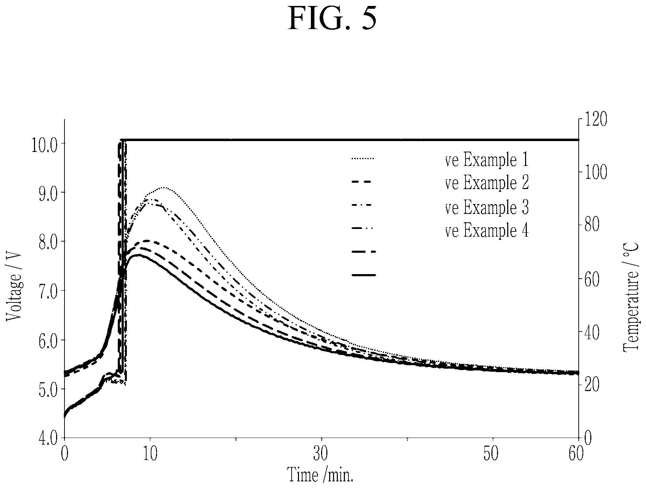

Overcharge evaluation is performed on the rechargeable lithium battery cells manufactured in Examples 1 to 18, and Comparative Examples 1 to 4 and the results are shown in Table 2 and FIG. 5.

After attaching a safety protection device to a negative electrode of each of the rechargeable lithium battery cells and a tab to a positive electrode by welding, a thermocouple is attached and fixed to the center of the cells to measure a temperature. Next, the cells are charged at a rate of 2.0 C to reach 10 V, and the maximum temperature (°C) of the cells during overcharge is measured.

Referring to Table 2, in Comparative Examples 1, 3, and 4 where the first compound is not added to the electrolyte solution, the maximum temperatures of the cells during overcharge are substantially high.

Also, referring to FIG. 5, in the case of Examples 1 and 2, the maximum temperatures of the cells reach about 70 °C before about 10 minutes have passed, whereas in the case of Comparative Examples 1, 3, and 4, the maximum temperatures of the cells reach about 88 °C or higher after 10 minutes.

### Evaluation Example 2: Evaluation of Heat Exposure Safety

The rechargeable lithium battery cells according to Examples 1 to 18 and Comparative Examples 1 to 4, which are in a discharge state of 2.8 V, are constant current-charged at a charge speed of 0.5 C under a cut-off condition of 4.2 V/3 hr and while maintaining the constant voltage of 4.2 V, charged to 0.05 C. After the charge, the fully charged cells to SOC 100 are subjected to a heat exposure evaluation.

The rechargeable lithium battery cells according to Examples 1 to 18 and Comparative Examples 1 to 4 are placed in a chamber and examined with respect to battery changes by increasing a temperature from room temperature (25 °C) to 139 °C, 140 °C, 141 °C, 142 °C, 143 °C, and 144 °C at 5 °C/min and maintaining each temperature for 1 hour.

After conducting the experiment three times in total, when no thermal runaway occurs, while maintaining each temperature, `P (Pass)' is given, but when rapid thermal runaway occurs, when exposed to each high temperature, `F (Fail)' is given, and the results are shown in Table 2.

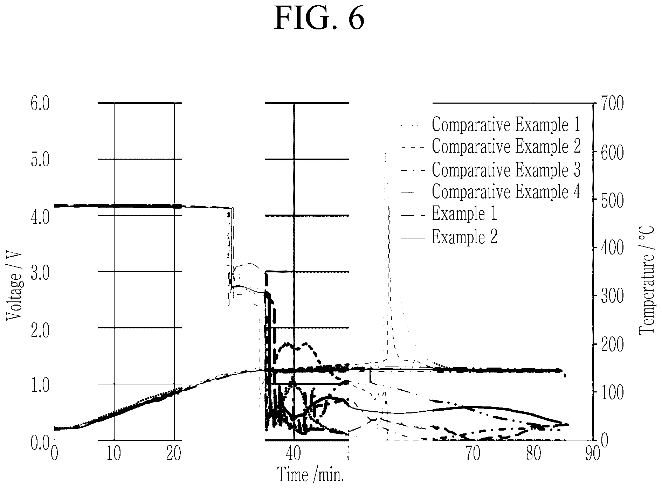

In addition, the thermal exposure results of the rechargeable lithium battery cells according to Examples 1 to 2 and Comparative Examples 1 to 4 are shown as a graph in FIG. 6. In FIG. 6, a relatively thick line represents a voltage change over time, and a relatively thin line represents a temperature change over time.

Referring to Table 2, Comparative Examples 1 and 2 in which the second compound is not added to an electrolyte solution, exhibit thermal runaway, when exposed to heat at 140 °C.

Referring to FIG. 6, the rechargeable lithium battery cells according to Examples 1 to 2 and Comparative Examples 1 to 4 exhibit a sharp voltage drop. When cylindrical batteries are suddenly exposed to a high temperature, gas is generated therefrom and increases an internal pressure, which activates a current interrupt device (CID), resulting in a sharp voltage drop. The sharp voltage drop of the rechargeable lithium battery cells of Examples 1 to 2 and Comparative Examples 1 to 4 confirms that the current interrupt device (CID) is activated due to gas generation caused by exposure to the high temperature.

For example, in the rechargeable lithium battery cells of Comparative Examples 1 and 2, as a vent thereof is completely opened at about 55 minutes, a voltage completely reaches 0 V. On the other hand, in the rechargeable lithium battery cells of Examples 1 to 2 and Comparative Examples 3 to 4 including both of the first and second compounds, the current interrupt device (CID) is activated, but the vent is not opened.

Referring to FIG. 6, the rechargeable lithium battery cells of Examples 1 and 2 are confirmed that even when exposed to a temperature of 142 °C, no thermal runaway occurs, while maintaining the temperature at 142 °C.

On the other hand, Comparative Example 1 exhibits rapid thermal runaway to about 500 °C at about 55 minutes, but Comparative Example 2 exhibits rapid thermal runaway to about 600 °C or more at about 56 minutes.

### Evaluation Example 3: Evaluation of DC-IR Resistance

The rechargeable lithium battery cells of Examples 1 to 18 and Comparative Examples 1 to 4 are measured with respect to direct current-internal resistance (DC-IR) in a state of charge (SOC = 100%), and the results are shown in Table 2.

Referring to Table 2 below, the rechargeable lithium battery cells of Examples 1 to 18 are conformed to exhibit desired or improved safety during overcharge and thermal exposure, as described above, and to maintain equivalent direct current-internal resistance (DC-IR) to those of the comparative examples.

**Table 2**

| | Overcharge maximum temperature (°C) | Heat exposure | | | | | | DC-IR (mΩ) |
|---|---|---|---|---|---|---|---|---|
| | | 139 °C | 140° C | 141 °C | 142 °C | 143 °C | 144 °C | |
| Comparative Example 1 | 94.1 | 3P/3 | 3F/3 | - | - | - | - | 25.88 |
| Comparative Example 2 | 74.0 | 3P/3 | 3F/3 | - | - | - | - | 26.82 |
| Comparative Example 3 | 89.7 | - | - | 3P/3 | 3P/3 | 3F/3 | - | 26.24 |
| Comparative Example 4 | 88.3 | - | - | - | 3P/3 | 3F/3 | - | 26.22 |
| Example 1 | 71.6 | - | - | - | 3P/3 | 3F/3 | - | 27.03 |
| Example 2 | 68.9 | - | - | - | 3P/3 | 3F/3 | - | 26.99 |
| Example 3 | 84.3 | - | - | - | 3P/3 | 3F/3 | - | 26.32 |
| Example 4 | 79.7 | - | - | - | 3P/3 | 3F/3 | - | 26.48 |
| Example 5 | 58.1 | - | - | - | 3P/3 | 3F/3 | - | 28.12 |
| Example 6 | 49.5 | - | - | - | 3P/3 | 3F/3 | - | 30.26 |
| Example 7 | 85.6 | - | - | - | 3P/3 | 3F/3 | - | 26.34 |
| Example 8 | 76.6 | - | - | - | 3P/3 | 3F/3 | - | 26.41 |
| Example 9 | 57.2 | - | - | - | 3P/3 | 3F/3 | - | 28.26 |
| Example 10 | 48.3 | - | - | - | 3P/3 | 3F/3 | - | 30.06 |
| Example 11 | 73.6 | 3P/3 | 3F/3 | - | - | - | - | 26.38 |
| Example 12 | 73.2 | 3P/3 | 3P/3 | 2F/3 | - | - | - | 26.93 |
| Example 13 | 70.3 | - | - | - | - | - | 3P/3 | 28.46 |
| Example 14 | 69.7 | - | - | - | - | - | 3P/3 | 30.44 |
| Example 15 | 71.6 | 3P/3 | 3F/3 | - | - | - | - | 26.84 |
| Example 16 | 70.9 | 3P/3 | 3P/3 | 1F/3 | - | - | - | 26.91 |
| Example 17 | 67.3 | - | - | - | - | - | 3P/3 | 28.39 |
| Example 18 | 66.9 | - | - | - | - | - | 3P/3 | 30.37 |

### Description of Symbols:

100: rechargeable lithium battery 10: positive electrode
11: positive electrode lead tab 12: positive terminal
20: negative electrode 21: negative electrode lead tab
22: negative terminal 30: separator
40: electrode assembly 50: case
60: sealing member 70: electrode tab
71: positive electrode tab 72: negative electrode tab

## Claims

1. An electrolyte solution for a rechargeable lithium battery, the electrolyte solution comprising:
a non-aqueous organic solvent;
a lithium salt; and
an additive,
wherein the additive comprises a first compound represented by Chemical Formula 1, and a second compound represented by Chemical Formula 2: 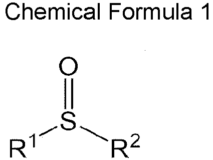 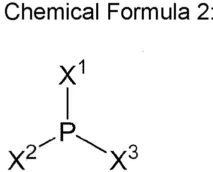
wherein, in Chemical Formula 1, R¹ and R² are each independently a substituted or unsubstituted C1 to C20 alkyl group or a substituted or unsubstituted C6 to C30 aryl group, provided that at least one of R¹ and R² is a substituted or unsubstituted C6 to C30 aryl group,
in Chemical Formula 2, X¹ to X³ are each independently a halogen or -O-L^{a}-R^{a},
at least one of X¹ to X³ is -O-L^{a}-R^{a},
L^{a} is each independently a single bond or a substituted or unsubstituted C1 to C10 alkylene group,
R^{a} is each independently a cyano group (-CN), a difluorophosphite group (-OPF₂), a substituted or unsubstituted C1 to C10 alkyl group, a substituted or unsubstituted C2 to C10 alkenyl group, a substituted or unsubstituted C2 to C10 alkynyl group, a substituted or unsubstituted C3 to C10 cycloalkyl group, a substituted or unsubstituted C3 to C10 cycloalkenyl group, a substituted or unsubstituted C3 to C10 cycloalkynyl group, or a substituted or unsubstituted C6 to C20 aryl group, and
R^{a} is each independently present, or at least two R^{a}s are linked to form a substituted or unsubstituted monocyclic or polycyclic aliphatic heterocycle, or a substituted or unsubstituted monocyclic or polycyclic aromatic heterocycle,
wherein "substituted" refers to replacement of at least one hydrogen of a substituent by deuterium, a halogen, a hydroxyl group, an amino group, a C1 to C30 amine group, a nitro group, a C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 fluoroalkyl group, or a cyano group.

2. The electrolyte solution as claimed in claim 1, wherein Chemical Formula 1 is represented by Chemical Formula 1-1 or Chemical Formula 1-2: 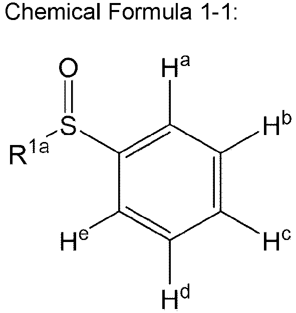
wherein, in Chemical Formula 1-1,
R^{1a} is a substituted or unsubstituted C1 to C20 alkyl group, and
H^{a} to H^{e} are each independently hydrogen, a halogen, a hydroxyl group, a cyano group, a nitro group, a substituted or unsubstituted C1 to C20 alkyl group, a substituted or unsubstituted C1 to C20 alkoxy group, a substituted or unsubstituted C2 to C20 alkenyl group, a substituted or unsubstituted C2 to C20 alkynyl group, a substituted or unsubstituted C3 to C20 cycloalkyl group, a substituted or unsubstituted C6 to C20 aryl group, or a substituted or unsubstituted C2 to C20 heteroaryl group; 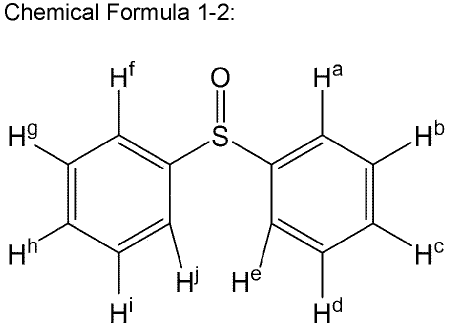
wherein, in Chemical Formula 1-2,
H^{a} to H^{j} are each independently hydrogen, a halogen, a hydroxyl group, a cyano group, a nitro group, a substituted or unsubstituted C1 to C20 alkyl group, a substituted or unsubstituted C1 to C20 alkoxy group, a substituted or unsubstituted C2 to C20 alkenyl group, a substituted or unsubstituted C2 to C20 alkynyl group, a substituted or unsubstituted C3 to C20 cycloalkyl group, a substituted or unsubstituted C6 to C20 aryl group, or a substituted or unsubstituted C2 to C20 heteroaryl group.

3. The electrolyte solution as claimed in claim 1, wherein the first compound is any one or more selected from the compounds listed in Group 1:

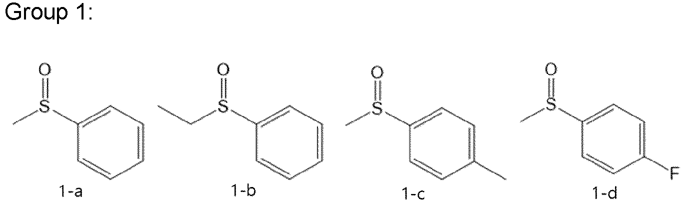

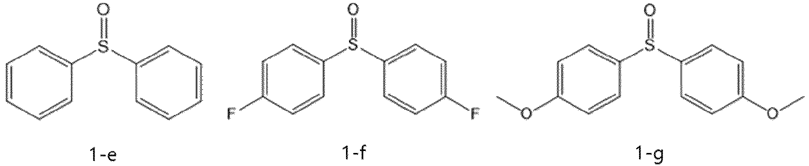

4. The electrolyte solution as claimed in any one of the preceding claims, wherein Chemical Formula 2 comprises one or more of the compounds represented by Chemical Formula 2-1 to Chemical Formula 2-3: 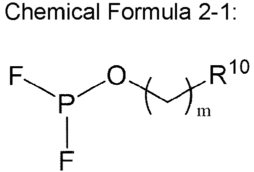
wherein, in Chemical Formula 2-1,
m is an integer in a range of 1 to 5, and
R¹⁰ is a cyano group (-CN) or a difluorophosphite group (-OPF₂); 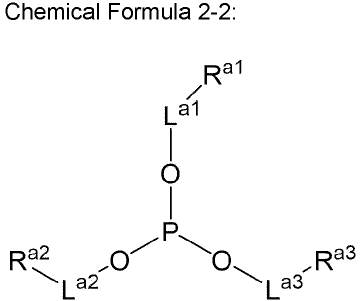
wherein, in Chemical Formula 2-2,
L^{a1} to L^{a3} are each independently a single bond or a substituted or unsubstituted C1 to C5 alkylene group, and
R^{a1} to R^{a3} are each independently a substituted or unsubstituted C1 to C10 alkyl group, a substituted or unsubstituted C2 to C10 alkenyl group, a substituted or unsubstituted C2 to C10 alkynyl group, a substituted or unsubstituted C3 to C10 cycloalkyl group, a substituted or unsubstituted C3 to C10 cycloalkenyl group, a substituted or unsubstituted C3 to C10 cycloalkynyl group, or a substituted or unsubstituted C6 to C20 aryl group; 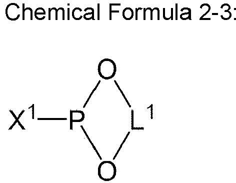
wherein, in Chemical Formula 2-3,
X¹ is a halogen or -O-L^{a4}-R^{a4},
L^{a4} is a single bond or a substituted or unsubstituted C1 to C5 alkylene group,
R^{a4} is a substituted or unsubstituted C1 to C10 alkyl group, a substituted or unsubstituted C2 to C10 alkenyl group, a substituted or unsubstituted C2 to C10 alkynyl group, a substituted or unsubstituted C3 to C10 cycloalkyl group, a substituted or unsubstituted C3 to C10 cycloalkenyl group, a substituted or unsubstituted C3 to C10 cycloalkynyl group, or a substituted or unsubstituted C6 to C20 aryl group, and
L¹ is a substituted or unsubstituted C2 to C5 alkylene group.

5. The electrolyte solution as claimed in claim 4, wherein the second compound is at least one of the compounds listed in Group 2:

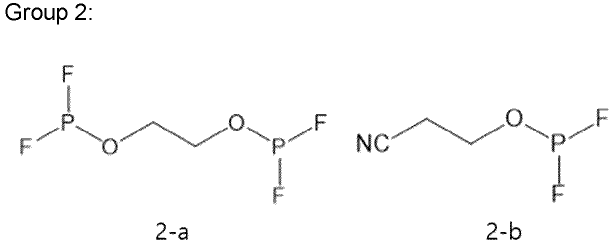

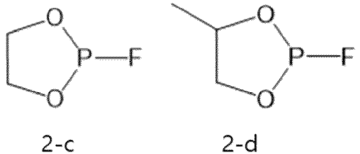

6. The electrolyte solution as claimed in any one of the preceding claims, wherein the first compound is included in an amount that is greater than 0.05 wt% and less than 6 wt% based on a total weight of the electrolyte solution for the rechargeable lithium battery.

7. The electrolyte solution as claimed in any one of the preceding claims, wherein the second compound is included in an amount that is greater than 0.05 wt% and less than 4 wt% based on a total weight of the electrolyte solution for the rechargeable lithium battery.

8. The electrolyte solution as claimed in any one of the preceding claims, wherein the first compound and the second compound are included in a weight ratio in a range of 0.01:1 to 100:1.

9. A rechargeable lithium battery (100), comprising:
a positive electrode (10) including a positive electrode active material;
a negative electrode (20) including a negative electrode active material;
a separator (30) between the positive electrode (10) and the negative electrode (20); and
the electrolyte solution as claimed in claim 1.
